Europäisches Patentamt

European Patent Office

Office européen des brevets

(11) Publication number: **0 439 210 A1**

# EUROPEAN PATENT APPLICATION

(21) Application number: 91200045.2

(22) Date of filing: 12.01.91

(51) Int. Cl.⁵: **G01N 33/569**, G01N 33/543, G01N 33/546

(30) Priority: 22.01.90 US 468392

(43) Date of publication of application:
31.07.91 Bulletin 91/31

(84) Designated Contracting States:
CH DE FR GB IT LI NL SE

(71) Applicant: EASTMAN KODAK COMPANY
343 State Street
Rochester, NY 14650-0221(US)

(72) Inventor: Snyder, Brian Anthony, c/o
EASTMAN KODAK COMPANY
Patent Department, 343 State Street
Rochester, New York 14650-2201(US)

(74) Representative: Nunney, Ronald Frederick
Adolphe et al
Kodak Limited Patent Department Headstone
Drive
Harrow Middlesex HA1 4TY(GB)

(54) **Differentiation of microorganisms associated with periodontal diseases , article and kit useful therein.**

(57) A method is provided for differentiation of the microorganisms associated with periodontal disease, such as Actinobacillus actinomycetemcomitans, Bacteroides gingivalis and Bacteroides intermedius, in a biological specimen. Thus, extracted antigens from any and all of the microorganisms are contacted with a microporous membrane having at least three discrete zones for immunological complexation. Antigens from each microorganism are complexed in specific zones so the operator can determine which microorganisms are present in the specimen by observing which zones have immunological complexes therein. Complexation occurs between the antigens and corresponding water-insoluble reagents which are composed of water-insoluble particles to which the appropriate antibodies are affixed. Detection of the complexes is achieved using labeled antibodies directed to the individual antigens. An article and kit useful in this method are also described.

FIG. 1

# DIFFERENTIATION OF MICROORGANISMS ASSOCIATED WITH PERIODONTAL DISEASES, ARTICLE AND KIT USEFUL THEREIN

This invention relates to a method for differentiating among microorganisms associated with periodontal diseases, such as the three microorganisms Actinobacillus actinomycetemcomitans, Bacteroides gingivalis and Bacteroides intermedius, in a biological specimen. It also relates to a water-insoluble article and kit useful in this method. This invention is useful in dental research and health care.

There is a continuing need in medical, dental and veterinary practices, in research and diagnostic procedures, and for rapid and accurate detection or quantification of biological or chemical substances present in biological fluids or specimens. For example, the presence of various microorganisms in human and animal tissues, fluids or cells is very important for diagnosis and effective treatment of diseases.

Specific microorganisms have been implicated as indicators for a number of periodontal diseases in humans and animals, such as gingivitis and periodontitis. The importance of such diseases is growing in the human population, especially as people live longer, and prevention thereof is becoming of considerable medical and commercial importance. In addition, the proper care of animals (including dental care) is a growing concern in our culture.

Detection of microorganisms associated with periodontal diseases has been accomplished using culture techniques (which are generally slow), DNA probes and a number of immunological procedures, such as agglutination assays, enzyme linked immunosorbent assays (ELISA), immunofluorescence and immunoprecipitation. The immunological procedures use immunological reactions of an antigenic site of the microorganism (which may be on a component extracted therefrom) with a corresponding antibody specific thereto. The resulting immunological reaction complex can then be detected in a number of ways.

Actinobacillus actinomycetemcomitans (identified herein as A. actinomycetemcomitans) is an oral gram-negative facultative organism which is closely related to the Haemophilus group of organisms. This organism can cause severe human infections, including bacterial endocarditis, thyroid gland abscess, urinary tract infection, brain abscess and vertebral osteomyelitis. It has been strongly implicated in the pathogenesis of certain types of periodontal disease, particularly in localized juvenile periodontitis and also in some forms of adult periodontitis. Human isolates of A. actinomycetemcomitans have been divided into three major serogroups designated as serotypes A, B and C.

The black-pigmented Bacteroides species are gram negative, anaerobic rods which are common in the etiology of various periodontal diseases, odontogenic abscesses and endodontic lesions. Such species include Bacteroides intermedius and Bacteroides gingivalis. As defined herein, periodontal disease refers to a wide variety of diseases in humans and animals which occur in the periodontal area of the oral cavity, and includes diseases affecting the connective tissue, loss of alveolar bone as well as the gingiva. It includes diseases that may occur at different times of human life, such as in infancy, youth, pregnancy or old age. Bacteroides intermedius and Bacteroides gingivalis have been associated with certain periodontal diseases.

Clinical assays specific to the bacteria noted above in gingival crevicular fluid and subgingival dental plaque are useful in the diagnosis of periodontal disease, in evaluating the progress of periodontal treatment, and in determining the status of the patient at later dental examinations. The standard culture techniques used to identify such organisms are time consuming, expensive and require a high level of operator expertise. Such tests also may lack sufficient sensitivity for detection of low levels of organism due to the strict anaerobic conditions required during transport.

Various immunoassays noted above have been developed and used with some success. Particularly useful are radioimmunoprecipitation assays and ELISA tests. US-A-4,741,999 describes monoclonal antibodies to antigens of A. actinomycetemcomitans and their use in ELISA assays. While these tests provide improvements over the standard culture techniques, further improvements are needed to increase assay sensitivity to the desired organism while at the same time minimizing cross-reactivity with other organisms which are similar, such as those in the Haemophilus group.

Monoclonal and polyclonal antibodies to various Bacteroides species have been prepared for similar assays [see for example, Nakazawa and others, noted above, and Zambon and others, J.Periodon., 56-(Supp), pp. 32-40, 1985].

Generally, the assays of the art have been slow, tedious and directed to a single organism. In many cases, the assays are highly cross-reactive with related species, and thus have limited usefulness.

In EP Application Serial No. ＿＿＿＿＿ (corresponding to U.S.S.N. 468,034 filed January 22, 1990), a screening assay is described for detecting the presence of any of the three microorganisms Actinobacillus actinomycetemcomitans, Bacteroides gingivalis and Bacteroides intermedius. This screening method is highly useful for practitioners so they can initially determine if a patient needs periodontal treatment.

However, once screening has been done, and the results are found to be positive, it would be desirable to be able to determine which microorganisms are present so treatment can be prescribed specifically. Such treatment could then begin early. Presently, any differentiation would have to be carried out using the tedious culture or the desirable immunological methods described above.

The problems noted above are overcome with a method for the differentiation of microorganisms associated with periodontal diseases comprising the steps of:

A. contacting an aqueous specimen suspected of containing an antigen extracted from a plurality of microorganisms associated with periodontal diseases with a microporous membrane having thereon,

in each of a plurality of discrete and individual zones of a surface of the membrane, each zone being less than the total area of the surface and being capable of indicating the presence of one of the specific microorganisms, a water-insoluble reagent comprising water-insoluble particles having affixed thereto antibodies directed to at least one antigen of the specific microorganism,

to form, in each of the zones, a water-insoluble immunological complex between the antibodies in the zone and the corresponding extracted antigen on the membrane,

B. prior to, simultaneously with or subsequent to the contact in step A, contacting each extracted antigen with a detectably labeled, water-soluble antibody directed thereto so as to form one or more labeled, water-insoluble immunological complexes specific for the microorganisms, the complexes being formed from both the labeled and water-insoluble antibodies with the respective extracted antigens in the respective zones on the membrane,

C. simultaneously with or subsequently to the contacting in step B, separating the labeled water-insoluble complexes from uncomplexed materials by washing the uncomplexed materials through the microporous membrane, and

D. detecting the labeled, water-insoluble complexes in the discrete and individual zones on the membrane as an indication of the presence or absence of at least one of the microorganisms in the specimen.

This invention also provides a water-insoluble article comprising a microporous membrane having at least first and second opposing outer surfaces, the article having affixed to each of a plurality of discrete and individual zones on at least one of the surfaces, a water-insoluble reagent,

the article characterized wherein each of the reagents comprises water-insoluble particles having affixed thereto antibodies directed to at least one antigen of a specific microorganism which is associated with periodontal diseases, each of the reagents being substantially all on the first or second surface.

A test kit for the differentiation of a plurality of microorganisms associated with periodontal diseases comprises:

a. a water-insoluble article comprising a microporous membrane having at least first and second opposing outer surfaces, and having affixed to each of a plurality of discrete and individual zones on at least one of the surfaces, a water-insoluble reagent, and

b. detectably labeled antibodies directed to each of the plurality of microorganisms, the antibodies being packaged individually or in admixture,

the kit characterized wherein each of the reagents comprises water-insoluble particles having affixed thereto antibodies directed to at least one antigen of a specific microorganism associated with periodontal diseases, each of the reagents being substantially all on the first or second surface.

The method of this invention is an effective, rapid and relatively inexpensive means for determining which of a plurality of microorganisms associated with periodontal diseases are present in a dental specimen. Thus, particular periodontal diseases can be more readily identified at earlier stages, and the appropriate treatment prescribed for early treatment. Prior to this time, such determinations were done by tedious culture methods, or insensitive immunological methods known in the art.

This method can be advantageously used after the screening test described in EP Application Serial No. _____ (corresponding to U.S.S.N. 468,034 noted above). That is, once a practitioner has quickly determined that at least one of the microorganisms exists in a specimen, the present invention can be used to find out which microorganisms are present. In some embodiments, the test can be designed to tell the practitioner which serotypes of microorganisms are present.

The noted advantages are achieved with a microporous membrane which has discrete zones for capturing immunological complexes of individual microorganisms or serotypes thereof. Each zone is designed for detecting only a specific microorganism by having the appropriate antibodies for that microorganism immobilized therein. Complexation of antigens and antibodies occurs rapidly on the membrane, and uncomplexed materials can be drained away leaving detectable complexes after contact with the appropriate labeled antibodies.

3

Bacteroides gingivalis is sometimes known as Porphyromonas gingivalis or as Bacteroides-(Porphyromonas) gingivalis.

FIG. 1 is a schematic plan view of an embodiment of this invention whereby three distinct circular regions of a membrane are outlined for detection of three specific microorganisms.

FIG. 2 is a schematic plan view of another embodiment of this invention whereby a membrane surface has nine distinct circular regions which can be designed for detecting different microorganisms, or detecting several serotypes of the same or different microorganisms.

FIG. 3 is a schematic plan view of still another embodiment of this invention whereby a membrane is shown with three distinct regions each in the shape of a "plus" sign.

The present invention can be used to rapidly and sensitively determine the presence of one or more microorganisms associated with periodontal diseases. In particular, the microorganisms Actinobacillus actinomycetemcomitans, Bacteroides gingivalis and Bacteroides intermedius can be differentiated by the present invention. However, other microorganisms which are suspected of being associated with periodontal diseases can also be detected and differentiated with this invention. Such other microorganisms include, but are not limited to, Wolinella recta, Bacteroides forsythus, Eikenella corrodens, Fusobacterium nucleoatum and Troponema denticola. In some embodiments, it is irrelevant as to which serotypes of any of the microorganisms may be present. In other embodiments, this method can be used to differentiate among serotypes of a single species of microorganism. This method is generally qualitative although the amount of complex observed in the detection technique may indicate a relative amount of the microorganisms. While the antigens detected with the method (lipopolysaccharides, capsule antigens or membrane proteins) may be part of intact cells, normally, they are extracted in a suitable manner from the cells present in a biological specimen. Such specimens include, but are not limited to, saliva or mucous from the throat or mouth, human or animal tissue extracts, dental plaque and gingival crevicular fluid. Generally, the organism is detected in dental plaque, saliva, or gingival crevicular fluid.

As noted above, this invention is a differentiation method, and it may be used alone as the only test in patient preventative care or treatment, but preferably, it is used after an initial screening which determines if any microorganisms are present. If the screening test is positive, then the present invention can be used to advantage to help the practitioner prescribe appropriate treatment or monitoring.

Antigen extraction from any of the microorganisms of interest is suitably accomplished using physical or chemical means, by use of a detergent (such as sodium deoxycholate, sodium dodecyl sulfate or sodium decyl sulfate) using standard procedures, as described, for example, in US-A-4,741,999, osmotic shock [see for example, Dirienzo and others, Infect. & Immun., 47(1), pp. 31-36, 1985], or sonic means [see for example, Zambon and others, Infect. & Immun., 41(1), pp. 19-27, 1983].

If desired, the antigenic material can be removed from the original specimen, or the original specimen can be suitably diluted with water or a suitable buffer, or filtered in order to remove extraneous matter and facilitate complexation of antigen and antibody in the assay.

Antibodies used in the practice of this invention can be monoclonal or polyclonal. Monoclonal antibodies can be prepared using standard procedures, such as those described in US-A-4,741,999. Polyclonal antibodies can also be produced using standard procedures, such as described, for example by Zambon and others, Infect. & Immun., 41(1), pp. 19-27 (1983). Generally, a mammal (such as a rabbit) is immunized one or more times with a suitable quantity of an antigenic component or whole bacterial cells of the organism. After a suitable time, when the titer is acceptable, antisera is recovered from the mammal. Antibodies can be removed from the antisera and purified if desired using known procedures and stored in frozen buffered solutions until used. Further details of such procedures are well known in the art.

Generally, once antibodies are isolated, they can be stored and used in buffered solutions containing one or more suitable buffers and a preservative if desired. Preferably, they are stored in frozen form. In the buffered solutions, the pH is generally maintained at from 6 to 9, and preferably at from 6.5 to 8.5. Useful buffers include phosphate buffered saline solution, tris(hydroxymethyl)aminomethane, tricine, bicine and other readily apparent to one skilled in the art.

The antibodies for each antigen can be stored and supplied separately for the assay or in a kit, or they can be mixed together for storage prior to usage. Since the specimen is being evaluated for the presence of a plurality of microorganisms (preferably, at least three microorganisms), a plurality of separate antibodies are used, each specific to only one microorganism. Because each of the microorganisms exists in more than one serotype, it is possible that antibodies to each serotype of a species would be used in admixture. One skilled in the art would be able to readily determine the number of antibodies to use, and the relative concentrations of each for adequate differentiation.

In the practice of the sandwich assay of this invention, antibodies are immobilized in a suitable fashion on water-insoluble, particulate carrier materials formed from organisms, polymers, glass, ceramics, natural

4

or synthetic resins, diatomaceous earth or magnetizable particles. These particles are preferably spherical in shape and have an average diameter of from 0.01 to 10 $\mu$m, preferably from 0.1 to 10 $\mu$m, and most preferably of from 1 to 3 $\mu$m, although the structural and spatial configurations are not critical as long as they are not entrapped within the membrane (described below). The average diameter may be designed so that the particles are retained on the membrane surface (as described below). The resulting water-insoluble reagent of antibody and particle is used in the method of this invention.

The antibodies can be affixed to particulate carrier materials by physical or chemical means, including adsorption or covalent reaction with reactive groups on the surface of the materials. Covalent attachment is preferred. Preferred polymeric particles have suitable surface reactive groups for covalently attaching the antibody molecules thereto. The density of the antibody molecules on the carrier materials may vary depending upon the composition of the materials, their size and the properties of antibody, but sufficient density needed for adequate sensitivity in the assay can be readily determined by one skilled in the art. The antibodies can be modified appropriately for attachment if necessary.

Covalent attachment of antibody is usually accomplished using surface reactive groups which are capable of reacting directly or indirectly (that is through linkages, such as proteins or avidin-biotin) with free amine or sulfhydryl groups of the antibody. Such surface reactive groups include, but are not limited to, carboxy, epoxy, aldehyde, active halo atoms, activated 2-substituted ethylsulfonyl, vinylsulfonyl and other groups known in the art. The following discussion regarding preferred embodiments is for exemplification only, and is not meant to be limiting.

Particularly useful polymeric particles include those described in EP-A-0 323 692. Such particles are generally water-insoluble latex particles having an average particle size greater than 0.01 micrometers. They are composed of polymers prepared from one or more ethylenically unsaturated polymerizable monomers at least one of which has active halo atoms, activated 2-substituted ethylsulfonyl or vinylsulfonyl groups. Monomers having reactive carboxy groups are also very useful.

One or more of the monomers described above can be polymerized individually or in combination to form homo- or copolymers. Alternatively, and preferably, one or more of them are copolymerized with at least one other ethylenically unsaturated polymerizable monomer. Generally such monomers provide various desirable properties such as hydrophobicity, dispersibility or other features. Particularly useful comonomers are described in EP-A-0 323 692.

Representative useful polymers include the following: poly(m & p-chloromethylstyrene), poly(styrene-co-m & p-chloromethylstyrene-co-2-hydroxyethyl acrylate) (67:30:3 molar ratio), poly[styrene-co-m & p-(2-chloroethylsulfonylmethyl)styrene] (96:4 molar ratio), poly{styrene-co-N[m & p-(2-chloroethylsulfonylmethyl)-phenyl]acrylamide} (99.3:0.7 molar ratio), poly(m & p-chloromethylstyrene-co-methacrylic acid)(95:5, 98:2 and 99.8:0.2 molar ratio), poly[styrene-co-m & p-(2-chloroethylsulfonylmethyl)styrene-co-methacrylic acid]-(93.5:4.5:2 molar ratio), poly{styrene-co-N-[m & p-(2-chloroethylsulfonylmethyl)phenyl]acrylamide-co-methacrylic acid)(97.3:0.7:2 molar ratio), and poly(styrene-co-m & p-chloromethylstyrene)(70:30 molar ratio).

Procedures for attachment of antibodies to particles are well known in the art. Representative procedures are described, for example, in EP-A-0 323 692, US-A-3,925,157, US-A-4,181,636, US-A-4,703,018, and other references too numerous to mention. In general, the antibodies are mixed with the particles under suitable conditions depending upon the attachment form (absorption, covalent or use of linking groups). A worker skilled in the art would readily determine the appropriate conditions from the art as well as exemplary teachings presented herein. For example, for attachment to particles having reactive halo atoms, activated 2-substituted ethylsulfonyl or vinylsulfonyl groups, the antibodies are generally mixed with the particles for up to 24 hours at a temperature of from 20° to 40°C in a suspension buffered at a pH of from 7 to 10. If carboxy groups are used for reaction, the well known carbodiimide activators can be used, as well as carbamoylonium activators such as those described in EP-A-0 308 235. Antibodies can be adsorbed onto particles by incubating particles and antibodies in suspension at suitable temperature for several hours.

An important aspect of this invention is the formation of an immunological complex of the extracted antigens and their antibodies in discrete and individual zones on a microporous membrane. Each zone is used to detect a specific microorganism, or a specific serotype of a specific microorganism. Contact of the antigens and all of the antibodies is substantially simultaneous so that all complexation between the individual antigens and their respective antibodies occurs around the same time. There may be a brief period of incubation at room temperature, or at a higher temperature, to allow sufficient complexation for detection.

The microporous membrane is water-insoluble and inert to any chemical or biological reactions. It is generally composed of one or more natural or synthetic substances which have sufficient integrity for reagents to react or be affixed thereto without loss of form or function. It is porous enough for filtration needed to remove substantially all uncomplexed materials from the complexes formed thereon. Useful membrane materials include, but are not limited to, porous natural or synthetic polymers, sintered glass, membranes of glass or polymeric films or fibers, ceramic materials, cellulosic materials and particulate structures composed of beads bound together with an adhesive or binder material. The membranes are generally flat, but some irregularities in the surfaces are acceptable, as well as some curvature if it is desired. One skilled in the art would be able to identify other useful materials which are commercially available or prepared using known techniques. Particularly useful materials are treated or untreated polyamide microporous membranes, such as those commercially available from Pall Corp.

The microporous membrane generally has an average pore size of from 0.5 to 10 $\mu$meters, although smaller or larger pores would be acceptable as long as the complexes formed remain on the outer surface, and drainage is not undesirably long.

The membrane is designed such that it has sufficient porosity to quickly drain away fluid and uncomplexed materials encountered during an assay. Such uncomplexed materials include uncomplexed antigen, antibodies, cellular debris and other nonparticulate extraneous matter from a biological specimen. Therefore, the pore size of the membrane must be such that the noted materials will pass through it.

The antibody-particle reagent is substantially on the surface of the membrane, meaning that less than 5% (by weight), and preferably less than 1% (by weight) of the reagent is entrapped within the membrane. By "entrapped" is meant that the entire reagent particle is within a pore of the membrane. This does not mean that reagent particles cannot be partially embedded in pores at or near the outer surface. Unintentionally, the microporous membrane may have some reagent partially embedded within its surface pores, but not a substantial amount like that taught in EP-A-0 200 381 and WO-A-87/03690.

If desired, the microporous membrane can be coated with proteins (such as casein or succinylated casein), as known in the art to reduce nonspecific interactions, or by surfactants to promote rapid filtration, or other optional materials which may facilitate the assay.

The individual particulate reagents having the antibodies specific to microorganisms are affixed to the membrane surface in any suitable manner as long as the antibodies are available for reaction with the contacting antigens. Generally, the reagents are affixed such that moderate mechanical disturbance during manufacture and handling does not loosen them. Moreover, the reagents generally do not come off the membrane during an assay. For example, the reagents can be affixed mechanically by coating, spotting or spraying and held thereon by hydrophobic bonding among molecules as well as between molecules and membrane. It can also be covalently affixed by suitable chemical reaction. Generally, the reagents are coated and dried on the membrane prior to use, although drying is not critical.

It is particularly useful that each reagent be immobilized on the membrane in admixture with a hydrophilic, neutral or positively-charged binder material. The amount of binder material is generally less than 20% based on the total weight of reagent.

Particularly useful binders include, but are not limited to, vinylpyrrolidone polymers, acrylamide polymers, and polymers containing quaternary salts and others known in the art which are either neutral in charge or positively charged. By "neutral in charge" is meant that either the polymer has no charges, or has both negative and positive charges which provide a net zero charge in the molecule. The polymers can be homo- or copolymers, and used singly or in combination. Acrylamide polymers are also defined to include methacrylamide polymers.

Useful positively-charged polymeric binders include mordants described in US-A-4,069,017 and references mentioned therein, provided that the mordants are sufficiently water-soluble. Sufficient water-solubility is generally provided by having at least 50% by weight of the polymer be composed of monomers containing the positively-charged groups.

The microporous membrane can be hand-held in the assay to provide sites thereon for complexation and filtration of the specimen into a separate container. However, preferably, the membrane is disposed or mounted in a water-insoluble article having a water-insoluble frame or structure for holding the membrane. Such articles are often identified in the art as "test devices", and many forms of such devices are known. Particularly useful devices are those described in US-A-3,825,410, US-A-3,888,629, US-A-3,970,429 and US-A-4,446,232 and those marketed as part of Surecell™ test kits (Eastman Kodak Co.) for detection of various antigenic material. Such devices are described in EP-A-0 308 231 and EP-A-0 321 261.

More specifically, the preferred test device comprises a water-insoluble shell having one or more test wells therein, each of which can accommodate a sample of a biological specimen and appropriate reagents.

The shell can be prepared from any useful water-insoluble material such as glass, polymeric materials, ceramics, fibrous materials, cellulosic materials and other materials known in the art.

In a preferred embodiment, the test device has three test wells designed for providing a specimen differentiation result and positive and negative control results. Each test well has a microporous membrane mounted therein. Other variations of useful test devices would be within the purview of a worker of ordinary skill in the art. In a preferred embodiment, one test well has a membrane on which the water-insoluble reagents are affixed in the desired zones for detecting each of the plurality of microorganisms individually.

The assay is carried out in a plurality of discrete zones of the membrane, each zone representing less than the total area of its surface. Such zones are provided by affixing the antibodies (as described below) to a region of at least one surface of the membrane, while leaving surrounding area free of antibodies. Preferably, each discrete zone is a round spot, although other configurations are within the purview of a skilled worker in the art. Preferably, there are at least three discrete zones on the membrane.

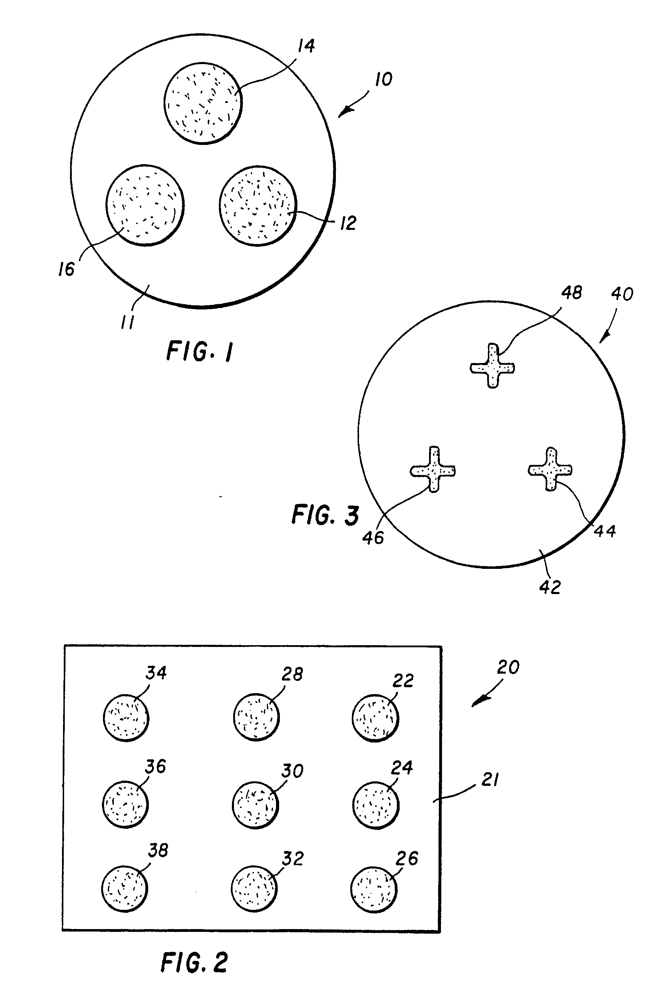

FIG. 1 is a schematic plan view of a preferred embodiment in which circular membrane 10 has upper surface 11 which comprises three distinct circular zones 12, 14 and 16. In each zone is a deposit of a water-insoluble reagent as described above. For example, zone 12 can have a reagent with antibodies directed to A. actinomycetemcomitans, zone 14 can have a reagent with antibodies directed to Bacteroides gingivalis and zone 16 can have a reagent with antibodies directed to Bacteroides intermedius.

FIG. 2 is a similar schematic illustration except that the design of membrane 20 is different than membrane 10 of FIG. 1. Membrane 20 includes upper surface 21 having nine distinct circular zones, 22, 24, 26, 28, 30, 32, 34, 36 and 38, each of which has a deposit of reagent with antibodies specific to a particular microorganism or microbial serotype. For example, zones 22, 24 and 26 can have reagents for detecting serotypes A, B and C, respectively of A. actinomycetemcomitans, zones 28, 30 and 32 can have reagents for detecting serotypes A, B and C of Bacteroides gingivalis and zones 34, 36 and 38, respectively, can have reagents for detecting serotypes A, B and C, respectively for Bacteroides intermedius.

FIG. 3 is still another embodiment where three distinct regions of water-insoluble reagent are deposited in the shape of "pluses" which show up during the assay if particular microorganisms are present in the specimen. If a microorganism is not present, only part of the "plus" symbol is visible (such as only the horizontal or vertical bar), or nothing shows up in the region specific therefor. Circular membrane 40 has upper surface 42 which includes three distinct regions 44, 46 and 48 for distinct microorganisms. A design alternative for this embodiment would be where each region has a different configuration, for example, an "A" for A. actinomycetemcomitans, an "I" for Bacteroides intermedius and a "G" for Bacteroides gingivalis.

In another design (unillustrated) of the membrane, the surface has a plurality (for example, three) distinct zones each of which has a reagent deposited therein which will give one detectable signal (for example a dye) for a negative determination of a microorganism, and a different detectable signal for a positive determination of a microorganism. The different signals can be from different dyes providing distinct colors, or different configurations of the same dye in different or the same regions.

The illustrations in FIGS. 1-3 are considered representative, and not limiting, of the various designs and formats of membranes with zones which can be devised for the practice of this invention.

The method of this invention is carried out in any fashion in which the antibodies on the membrane are contacted with the antigens extracted from the microorganisms in the specimen so that immunological complexes are formed between the antibodies and the respective antigens which are present. Contact can be accomplished in any suitable manner, but preferably, the specimen containing extracted antigen is dropped or poured onto the membrane, which may be in a disposable test device.

At some point in the assay, either before, after or simultaneously with the formation of one or more complexes between antigen and the antibodies of the respective particulate reagent, the antigen also forms an immunological complex with a second antibody directed thereto, which second antibody is water-soluble and detectably labeled. By "detectably labeled" is meant that the antibody has a moiety covalently attached thereto or otherwise associated therewith which can be directly or indirectly detected visually or by using spectophotometer, radiometric or other suitable equipment and procedures. Indirect detection can be accomplished by reacting the moiety on the antibody one or more times with suitable reagents to render the resulting product directly detectable.

Useful labels are well known and include enzymes, radioisotopes, specific binding materials such as biotin, avidin, lectins, sugars, and other materials readily apparent to one skilled in the art which can be detected in some manner. Preferably, the label is biotin, an enzyme or a radioisotope. Preferably, contact with the second antibody occurs after complex formation with the insolubilized antibody on the membrane. The resulting bound sandwich complex in each zone is thus formed of a specific antigen and two antibodies directed to different epitopic sites thereof, one antibody insolubilized, the second antibody being labeled for detection.

The methods for preparation of labeled antibodies are well known. Enzymes, such as peroxidase, glucose oxidase, alkaline phosphatase, urease, $\beta$-galactosidase and glucosidase are particularly useful labels. Peroxidase is a most preferred label.

Simultaneously or subsequently to the contact of the labeled antibody with the antigen, uncomplexed materials are separated from the sandwich complex bound to the membrane by washing the uncomplexed

7

materials through the membrane. There may be sufficient fluid in the biological specimen to wash the materials through the membrane, but generally, one or more separate wash fluids are applied to accomplish separation. Wash fluids are well known in the art, and include distilled water or buffered solutions of nonionic surfactants. A particularly useful wash fluid includes sodium decyl sulfate or sodium dodecyl sulfate.

Detection of the sandwich complex on the membrane is accomplished using standard detection equipment and procedures. They will vary with the particular label as one skilled in the art would readily perceive. Where the label is an enzyme, the bound complex is further contacted with a composition which will provide a dye (chromogen or fluorogen) in the presence of the enzyme. Such as composition generally includes one or more reagents which are substrates for the enzyme. Such reagents are known to one skilled in the art.

Where peroxidase is the label, a number of suitable dye-forming compositions are known comprising a substrate or substrate-forming reactants as well as dye-forming reactants. The substrate itself can be a dye-forming compound, such as benzidine, tetramethylbenzidine or other benzidine derivatives, 2,2'-azino-di-(3-ethyl-benzthiazolone-6-sulfonic acid), phenol red, o-phenylenediamine, pyrogallol, 4-aminoantipyrine, bromopyrogallol red and others known in the art. Alternatively, a hydrogen donor and an electron acceptor can be combined to provide a detectable species as is known in the art.

Preferably, the dye-forming composition includes a leuco dye which provides a dye in the presence of hydrogen peroxide and peroxidase (for example, a triarylimidazole leuco dye as described in US-A-4,089,747 or a triarylmethane leuco dye as described in US-A-4,670,385). A preferred dye-providing composition is illustrated in the examples below.

Once a dye has been formed in the presence of the insoluble complex, it can be evaluated visually or by using spectrophotometric equipment to determine if the assay indicates the presence of antigen in the specimen. Both positive and negative control tests may be desirably carried out with the specimen test. Appropriate reagents could be used for each control test to give the desired result.

In one embodiment, where the label is biotin or avidin, detection can be achieved by reacting the label with the corresponding receptor (that is, biotin as the label reacted with avidin). The corresponding receptor can be suitable labeled with a radioisotope or enzyme to render the resulting complex detectable. For example, if biotin is the label, it can be reacted with a conjugate of an enzyme and avidin. The enzyme can produce a detectable dye in the presence of appropriate reagents.

In a preferred embodiment, a method for the differentiation of the microorganisms Actinobacillus actinomycetemcomitans, Bacteroides gingivalis and Bacteroides intermedius comprises the steps of:

A. extracting antigen from one or more serotypes of each of the microorganisms Actinobacillus actinomycetemcomitans, Bacteroides gingivalis and Bacteroides intermedius present in a biological specimen,

B. contacting all antigens extracted in step A with a microporous membrane in a disposable test device, the membrane having thereon,

in each of at least three discrete and individual zones of a surface of the membrane, each zone being less than the total area of the surface and being capable of indicating the presence of one of the microorganisms, a water-insoluble reagent comprising water-insoluble particles having affixed thereto antibodies directed to at least one antigen of the specific microorganisms,

to form, in each of the zones, a water-insoluble immunological complex between the antibodies in the zone and the corresponding to extracted antigen on the membrane,

C. contacting each water-insoluble complex on the membrane with an enzyme-labeled, water-soluble antibody directed to the antigen of each complex so as to form an enzyme-labeled, water-insoluble immunological complex of both the labeled and water-insoluble antibodies with the respective extracted antigen in the respective zone on the membrane,

D. washing uncomplexed materials through the membrane, and

E. contacting each enzyme-labeled complex on the membrane with a composition which provides a dye in the presence of the enzyme as an indication of the presence or absence of each microorganism in the specimen.

The diagnostic test kit of this invention includes the article of this invention as well as detectably labeled antibodies directed to each of the microorganisms that the article is designed to detect. These kit components can be packaged, individually or together, in a suitable manner and included in a carrier of some type which can be compartmentalized to hold the article (alone or in a test device) and vials or bottles of reagents. In addition, it can also include one or more of the following which are optionally useful in carrying out the screening method: dye-forming composition, extraction reagents, wash solutions, diluents, and other reagents known to one skilled in the art for a given assay format. Reagents can be provided in dry form or in appropriate solutions. Non-reactive components of the kit can include instructions, mixing vessels, stirring means, pipettes and the like.

The following preparation of polyclonal antibodies is a preferred method for their preparation.

The bacterial strains were supplied as viable cultures by Homer S. Reynolds (SUNY, Buffalo, New York School of Dentistry). Isolates were anaerobically cultured on CDC anaerobic plates (BBL Laboratories, Baltimore, Maryland) supplemented with hemin (5 $\mu$g/ml) and menadione (0.5 $\mu$g/ml).

The plates were then incubated for 24-48 hours at 37°C in an anaerobic chamber. Frozen stocks were prepared for each strain by harvesting the colonies with an inoculating loop, preparing a cell suspension in skim milk and freezing the suspension at -70°C. Viability of the frozen stock was tested by plating a portion of the frozen stock onto CDC anaerobic plates and incubating as described above. Once viability was determined, fresh isolates for each strain could be obtained in a similar manner. Cell suspensions were prepared by harvesting colonies with an inoculating loop, placing the loop in phosphate buffered saline solution (pH 7.5, 1 ml) and vortexing vigorously for about one minute.

Concentrations of cell suspensions were determined spectrophotometrically by measuring turbidity at 620 nm. Appropriate dilutions were prepared to yield an optical density in the range of 0.1 to 1. Concentrations were determined using a 1 McFarland standard wherein $OD_{620}$ = 0.180, corresponds to about 3 x $10^8$ cells/ml.

Ten New Zealand white rabbits were intravenously injected with 0.5 ml of a phosphate buffered saline solution (pH 7.3) solution of the respective immunogen containing about 5 x $10^8$ whole viable cells per ml. The injections were given following the method shown in McCarty and Lancefield (J.Exp.Med., 102, pp. 1-28, 1955).

Two booster injections were given as follows: one week after the initial injection, each rabbit was injected again with the same amount of immunogen, and one additional week later, each rabbit was similarly injected.

Beginning with the fifteenth day after the initial injection, for a total of eleven additional weeks, each rabbit was given a booster injection of 1 ml of immunogen (5 x $10^8$ cells/ml) three times per week. The booster injections were spaced out every other day over each seven day period. Samples (2 ml) of antisera (for each serotype) were collected from the rabbits at various times to determine the antibody performance at those times. For example, antisera samples were taken at three, six and nine week intervals after the initial injection of immunogen. The rabbits were then sacrificed at thirteen weeks after the initial injection and the antisera was collected (about 100 ml per rabbit).

The final sera were then purified using ammonium sulfate precipitation. Saturated ammonium sulfate solution was added dropwise with stirring to each serum sample, cooled on ice until 45% saturation was achieved. After the addition, the mixture was stirred for an additional ten minutes, centrifuged and the supernatant discarded. The pellet was resuspended in a volume of phosphate buffered saline solution (pH 7.3) equal to the amount of the original sample being purified. The noted steps (addition of ammonium sulfate, centrifugation and resuspension of the pellet) were repeated. The mixture was then transferred to dialysis bags and dialyzed for about fifteen hours at 4°C with stirring against phosphate buffered saline solution (pH 7.3). About 200-10,000 times excess of dialysis buffer was used. The dialysis was repeated with fresh buffer for an additional six hours. The solutions were removed from the dialysis bags and filtered through a 0.22 $\mu$meter filter. A portion of the filtrate was diluted in the range of 1:10 to 1:100 in phosphate buffered saline solution, and the absorbance was read at 280 nm. The antibody concentration was determined according to the formula:

$A_{280}$ _ [1.4 x (dilution)] = antibody conc.(mg/ml)

The antibody solutions were then diluted to 2-4 mg/ml with phosphate buffered saline solution (pH 7.3) and merthiolate (0.01 weight %) and stored at 4°C (for small amounts whereas large amounts were stored at -70°C).

The following examples are presented to illustrate the practice of the invention, but are not intended to be limiting in any way. All percentages are based on weight, unless otherwise indicated.

Examples 1-3 Sandwich Differentiation Assays

These examples illustrate the use of the present invention to simultaneously detect the presence of any of the microorganisms Actinobacillus actinomycetemcomitans, Bacteroides gingivalis and Bacteroides intermedius in a biological specimen using a single test device.

Materials and Methods:

Surecell™ disposable test devices were used containing LoProdyne™ nylon microporous membranes (5 μm, Pall Corp.) incorporated into the test wells. The membranes had been pretreated with Fluorad™ FC 135 nonionic surfactant (0.05 g/m², 3M Co.). The membranes were set into the test wells so that the bottom of each test well was 9 mm in diameter to provide sufficient surface area for each distinct region.

A dye-providing composition was prepared to include 2-(4-hydroxy-3,5-dimethoxyphenyl)-4,5-bis(4-methoxyphenyl)imidazole (0.008%), poly(vinylpyrrolidone) (1%), sodium phosphate buffer (10 mmolar, pH 6.8), hydrogen peroxide (10 mmolar), 4'-hydroxyacetanilide electron transfer agent (2 mmolar) and diethylenetriaminepentaacetic acid chelating agent (10 μmolar).

Polyclonal antibodies directed against each of the three microorganisms, Actinobacillus actinomycetemcomitans, Bacteroides gingivalis and Bacteroides intermedius, were obtained by intravenous injection of rabbits as described above. IgG fractions were prepared by ammonium sulfate precipitation, and stored at 4° C in phosphate buffered saline solution (0.3-0.4% solution). The bacterial strains used to produce the antisera were supplied as viable cultures by H.S. Reynolds (SUNY, Buffalo, New York School of Dentistry). Isolates were subcultured on anaerobic plates as described above. The microorganisms were those identified by deposit numbers of ATCC 43717 for Actinobacillus actinomycetemcomitans (serotype A), ATCC 53978 for Bacteroides gingivalis (serotype B) and ATCC 25611 for Bacteroides intermedius (serotype A).

Polymer-antibody reagents were prepared by covalently binding equal amounts of antibodies directed to A. actinomycetemcomitans, equal concentrations of serotype A (ATCC 43717) and serotype B (ATCC 43718) to polymeric particles of poly[styrene-co-m & p-(2-chloroethylsulfonylmethyl)styrene] (96:4 molar ratio) which had been made using the teaching of EP-A-0 323 692. Covalent attachment was achieved by adding the antibodies (56.5 μg in 67 μl of phosphate buffered saline solution) to a solution of borate buffer (659 μl, 0.05 molar, pH 8.5) in a test tube and mixed well. The polymeric particles (228 μl suspension containing 13.15% solids) were added to the antibody composition, and the resulting suspension was rotated end-over-end for 14-24 hours at room temperature to allow covalent attachment of antibodies to the particles. The suspension was then centrifuged at 2800 rpm for 10 minutes. The supernatant was discarded and the pellet was suspended in glycine buffer (1 ml, 0.01 molar, pH 8.5) containing merthiolate (0.01%). This procedure was repeated twice.

In the same manner, antibodies to Bacteroides intermedius, equal concentrations of serotype A (ATCC 25611), serotype B (NCTC 9336) and serotype C (ATCC 49046) were covalently bound to the same type of polymeric particles. Moreover, antibodies to Bacteroides gingivalis, equal concentrations of serotype A (ATCC 33277), serotype B (ATCC 53978) and serotype C (ATCC 53977) were similar bound to the same type of polymeric particles.

The final antibody/particle reagent composition for each microorganism contained the following: antibody/particle reagent (1%), polyacrylamide binder (5%) in glycine buffer (0.1 molar, pH 8.5). Each reagent composition (2 μl) was then applied to a distinct zone of the top of the LoProdyne™ nylon microporous membrane in the test devices and dried to form an article of the present invention. Thus, the membrane had three separate zones of antibody-particle reagents, each zone having a reagent specific to a particular microorganism.

Enzyme-antibody conjugates were prepared using antibodies directed to each microorganism, the antibodies conjugated to horseradish peroxidase using the procedure of Yoshitake and others (Eur. J. Biochem., 101, 395, 1979). Each conjugate composition comprised the conjugates (5 μg of each per ml), casein (0.5%) and merthiolate (0.01%) in 3-(N-morpholino)propanesulfonic acid buffer (0.1 molar, pH 7.5).

Antigens were extracted from the microorganisms using sodium dodecyl sulfate (10%) for about one minute at room temperature.

A wash solution comprised sodium decyl sulfate (18 g/l) in water.

The buffers mentioned and used herein are available from a number of commercial sources including Sigma Chemical Co. (St. Louis, Missouri).

The extracted antigen from each microorganism (50 μl), at two different concentrations, were added to two sets of test devices described above. The surface area of the membranes which had no antibody-particle reagent thereon were used as negative controls for each test well.

The conjugate composition (50 μl) was added and the test devices were incubated at room temperature for 5 minutes. The wash solution (about 500 μl) was added one drop at a time to each test well. The dye-providing composition (50 μl) was added and the test devices incubated at room temperature for 2 minutes. The dye formed on the membranes was visually read and compared to a calibrated color chart having reflectance density values, then converted to transmittance density ($D_T$) using the Williams-Clapper transform (J. Opt. Soc. Amer., 43, 595, 1953).

The results of the assays are shown in the following Table. Example 1 is used to identify detection of A.

actinomycetemcomitans with the antibody-particle reagent having antibodies directed to that microorganism on the membrane. Example 2 identifies the detection of Bacteroides gingivalis using the reagent specific thereto on the membrane. Example 3 identifies the detection of Bacteroides intermedius using the reagent specific thereto on the membrane.

The results indicate that each microorganism is differentiated from the other microorganisms in the sandwich assay performed in a single test well.

T A B L E

| Antigen Concentration (Cells) | $D_T$ | | | |
| --- | --- | --- | --- | --- |
| | Example 1 Reagent | Example 2 Reagent | Example 3 Reagent | Negative Control |
| Example 1: Detection of A. actinomycetemcomitans | | | | |
| $3.13 \times 10^6$ * | 0.195 | 0.021 | 0.021 | 0.021 |
| $1.95 \times 10^5$ * | 0.114 | 0.021 | 0.021 | 0.021 |
| Example 2: Detection of B. gingivalis | | | | |
| $6.25 \times 10^6$ @ | 0.021 | 0.145 | 0.021 | 0.021 |
| $7.81 \times 10^5$ @ | 0.021 | 0.101 | 0.021 | 0.021 |
| Example 3: Detection of B. intermedius | | | | |
| $6.25 \times 10^6$ # | 0.021 | 0.021 | 0.175 | 0.021 |
| $7.81 \times 10^5$ # | 0.021 | 0.021 | 0.073 | 0.021 |

In the Table above, * identifies cells of Actinobacillus actinomycetemcomitans, @ identifies cells of Bacteroides gingivalis and # identifies cells of Bacteroides intermedius.

## Claims

1. A method for the differentiation of microorganisms associated with periodontal diseases comprising the steps of:

A. contacting an aqueous specimen suspected of containing an antigen extracted from a plurality of microorganisms associated with periodontal diseases with a microporous membrane having thereon, in each of a plurality of discrete and individual zones of a surface of said membrane, each zone being less than the total area of said surface and being capable of indicating the presence of one of said microorganisms, a water-insoluble reagent comprising water-insoluble particles having affixed thereto antibodies directed to at least one antigen of said specific microorganism,

to form, in each of said zones, a water-insoluble immunological complex between said antibodies in the zone and the corresponding extracted antigen on said membrane,

B. prior to, simultaneously with or subsequent to the contact in step A, contacting each extracted antigen with a detectably labeled, water-soluble antibody directed thereto so as to form one or more labeled, water-insoluble immunological complexes specific for said microorganisms, the complexes being formed from both said labeled and water-insoluble antibodies with the respective extracted antigens in the respective zones on the membrane,

C. simultaneously with or subsequently to the contacting in step B, separating said labeled water-insoluble complexes from uncomplexed materials by washing the uncomplexed materials through said microporous membrane, and

D. detecting said labeled, water-insoluble complexes in said discrete and individual zones on said membrane as an indication of the presence or absence of at least one of said microorganisms in said specimen.

2. The method as claimed in claim 1 wherein the water-soluble antibodies are labeled with an enzyme, radioisotope or biotin.

3. The method as claimed in either of claims 1 and 2 for the detection of a lipopolysaccharide or capsule antigen of each microorganism.

4. A water-insoluble article comprising a microporous membrane having at least first and second opposing outer surfaces, and having affixed to each of a plurality of discrete and individual zones on at least one of the surfaces, a water-insoluble reagent,

the article characterized wherein each of the reagents comprises water-insoluble particles having affixed thereto antibodies directed to at least one antigen of a specific microorganism which is associated with periodontal disease, each of the reagents being substantially all on the first or second surface.

5. The article as claimed in claim 4 wherein less than 1%, by weight, of each reagent is entrapped by the membrane.

6. A test kit for the differentiation of a plurality of microorganisms associated with periodontal disease, comprising:

a. a water-insoluble article comprising a microporous membrane having at least first and second opposing outer surfaces, and having affixed to each of at least three discrete and individual zones on at least one of the surfaces, a water-insoluble reagent, and

b. detectably labeled antibodies directed to each of the plurality of microorganisms, the antibodies being packaged individually or in admixture,

the kit characterized wherein each of the reagents comprises water-insoluble particles having affixed thereto antibodies directed to at least one antigen of a specific microorganism associated with periodontal disease, each of the reagents being substantially all on the first or second surface.

7. The kit as claimed in claim 6 wherein the antibodies of (b) are labeled with an enzyme, and the kit further comprises a composition which provides a dye in the presence of the enzyme.

8. The invention as claimed in any of claims 1 to 7 for the differentiation among Actinobacillus actinomycetemcomitans, Bacteroides intermedius and Bacteroides gingivalis.

9. The invention as claimed in claim 8 for the detection of all serotypes of each microorganism in three discrete and individual zones.

10. The invention as claimed in any of claims 1 to 9 wherein the membrane has a pore size of from 0.5 to 10 $\mu$meter, and the water-insoluble particles have an average diameter of from 0.01 to 10 $\mu$meters.

11. The invention as claimed in any of claims 1 to 10 wherein the antibodies are covalently attached to the particles.

12. The invention as claimed in any of claims 1 to 11 for the differentiation of each serotype of each microorganism, wherein each microbial serotype is detected in a discrete zone of the membrane which is less than its total surface area using a water-insoluble reagent specific for each serotype.

13. The invention as claimed in any of claims 1 to 12 wherein each reagent is affixed to the membrane in admixture with a hydrophilic neutral or positively-charged binder material.

12

FIG. I

FIG. 3

FIG. 2

European
Patent Office

EUROPEAN SEARCH
REPORT

Application Number

EP 91 20 0045

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 269 388 (MINNESOTA MINING AND MANUFAC-TURING CO.) <br> * Page 3, lines 63-64; page 13, examples 6,7; page 15, example 9 * | 1-13 | G <br> 01 N 33/569 <br> G 01 N 33/543 <br> G 01 N 33/546 |
| Y | EP-A-0 253 464 (HYBRITECH INC.) <br> * Page 8, lines 22-29; figure 3 * | 1-13 | |
| T | JOURNAL OF DENTAL RESEARCH, vol. 69, special issue March 1990, page 243, abstract no. 1077, Eastman Kodak Co., Rochester, NY, US; P. CONTESTABLE et al.: "A rapid enzyme immunoassay for three suspected periodontal pathogens" <br> * Whole abstract * | 1-13 | |
| A | EP-A-0 335 244 (ABBOTT LABORATORIES) <br> * Page 5, line 1 - page 6, line 40; page 8, lines 20-37 * | 10,11 | |

TECHNICAL FIELDS
SEARCHED (Int. Cl.5)

G 01 N

The present search report has been drawn up for all claims

| Place of search | Date of completion of search | Examiner |
|---|---|---|
| The Hague | 18 April 91 | VAN BOHEMEN C.G. |